# EUROPEAN PATENT APPLICATION

(11) **EP 3 842 019 A1**
(43) Date of publication of application: **30.06.2021**
(21) Application number: 19852694.9
(22) Date of filing: 05.08.2019
(51) Int. Cl.: A61F 13/537, D04H 1/425, D04H 1/4374, D04H 1/4382

(54) **ABSORBENT ARTICLE**

(30) Priority: 22.08.2018 JP 2018155288
(71) Applicant: Daio Paper Corporation, Ehime 799-0492 (JP)
(72) Inventor: OKADA, Yuki, Shikokuchuo-shi, Ehime 799-0431 (JP); FURUKAWA, Masashi, Shikokuchuo-shi, Ehime 799-0431 (JP)
(74) Representative: Williams Powell
(86) International application number: PCT/JP2019/030678
(87) International publication number: WO 2020/039905

(57) **Abstract**

To improve the diffusibility of excreted liquid by a simple method.

An absorbent article according to the present invention includes a liquid pervious top sheet 30 that includes a portion located on a surface, an absorber 56 that contains a super absorbent polymer provided on a back surface side of the top sheet 30, and a liquid pervious intermediate sheet 40 provided between the top sheet 30 and the absorber 56. The top sheet 30, the intermediate sheet 40, and the absorber 56 are provided at least in a region from a first position in a crotch portion or in a vicinity of the crotch portion to a second position apart from the first position, and the intermediate sheet 40 has an adhered portion of a cellulose nanofiber assembly continuing over at least the first position and the second position.

## Description

### Technical Field

The present invention relates to a disposable absorbent article such as an underpants-type diaper, a tape-type diaper, and a sanitary napkin.

### Background Art

The absorbent article includes a top sheet that forms a surface and an absorber provided on the back surface side of the top sheet, and excreted liquid excreted on the top sheet is supplied to the absorber through the top sheet and is basically absorbed and held by the absorber. As is well known, the absorber generally contains a super absorbent polymer.

One of the typical absorption performances in such an absorbent article is diffusibility in a direction perpendicular to a thickness direction. Generally, when the diffusibility is low, it is necessary to hold excreted liquid in a part of an absorber, but since the absorption speed of a super absorbent polymer is limited, if pressure is applied in the thickness direction before the super absorbent polymer has completely absorbed the excreted liquid, the excreted liquid that has moved to the absorber returns to a top sheet again, and there is a possibility that a phenomenon of sticking to the skin (hereinafter, also referred to as returning) may occur.

Various means are known as means for improving the diffusibility, such as providing a groove along the diffusion direction in the absorber (refer to, for example, Patent Literature 1).

### Citation List

### Patent Literature

Patent Literature 1: JP 2017-140251 A
Patent Literature 2: JP 5502742 B2

### Summary of Invention

### Technical Problem

However, most of the conventional diffusibility improving means are accompanied by major design changes such as structural changes, and it is difficult to improve the diffusibility while utilizing the existing design.

Therefore, a main object of the present invention is to improve the diffusibility of excreted liquid by a simple method.

### Solution to Problem

The absorbent article which solved the above-described subject is as follows.

### <First Aspect>

An absorbent article, includes
a liquid pervious top sheet including a portion located on a front surface,
an absorber containing a super absorbent polymer provided on a back surface side of the top sheet, and
a liquid pervious intermediate sheet provided between the top sheet and the absorber,
in which the top sheet, the intermediate sheet, and the absorber are provided at least in a region from a first position in a crotch portion or in a vicinity of the crotch portion and a second position apart from the first position,
the intermediate sheet has an adhered portion of a cellulose nanofiber assembly continuing at least from the first position to the second position.

### (Function and Effect)

The cellulose nanofiber assembly is obtained by applying a dispersion liquid of cellulose nanofibers to an object to be adhered and then drying. As a result of earnest research, the present inventors have found that, when the cellulose nanofiber assembly is attached (fixed) to the intermediate sheet of the absorbent article, the diffusibility is improved. That is, since the cellulose nanofiber assembly has high hydrophilicity, excreted liquid in contact with the intermediate sheet in the absorbent article is promoted so as to diffuse in the direction following the adhered portion of the cellulose nanofiber assembly (that is, from the first position to the second position). Therefore, it is possible to improve the diffusibility by a simple method of applying the cellulose nanofiber dispersion liquid without (or with) a major design change such as a structure change. As a result, in the present absorbent article, the absorption speed is improved due to the improvement of the diffusibility outside the absorber, such that the returning phenomenon is less likely to occur.

Incidentally, Patent Literature 2 describes an invention that utilize cellulose nanofibers in an absorbent article, but this relates to a water resistant and highly air permeable composite sheet, and does not relate to the diffusibility of excreted liquid in the intermediate sheet.

### <Second Aspect>

In the absorbent article according to the first aspect,
the intermediate sheet is a nonwoven fabric, and the adhered portion of the cellulose nanofiber assembly includes coated fibers, in which a fiber surface of a nonwoven fabric is coated with the cellulose nanofiber assembly, and a gap between the coated fibers.

### (Function and Effect)

The nonwoven fabrics are suitable as the intermediate sheet, and in this case, the adhered portion of the cellulose nanofiber assembly has coated fibers in which the fiber surface of the nonwoven fabric is coated with the cellulose nanofiber assembly, and a gap between the coated fibers, such that, without impairing the liquid perviousness, the diffusibility can be improved microscopically along individual fibers and macroscopically in the direction in which the adhered portion continues. In particular, the high density and high hydrophilicity of the surface of each coated fiber improve the diffusibility of excreted liquid along the fiber.

### <Third Aspect>

In the absorbent article according to the second aspect,
the intermediate sheet contains the cellulose nanofiber assembly on the surface of the absorber side and inside the absorber, and
the content of the cellulose nanofiber assembly in the intermediate sheet is decreasing from the absorber side in the intermediate sheet toward the top sheet side.

### (Function and Effect)

In the cellulose nanofiber assembly, the diffusibility is improved as the area is increased, but the intermediate sheet is hardened. Therefore, as in the present aspect, when the content of the adhered portion of the cellulose nanofiber assembly decreases from the absorber side in the intermediate sheet toward the top sheet side, this makes it possible to suppress the hardening of the wearer's touch feeling due to the cellulose nanofiber assembly. Further, by relatively increasing the content of the cellulose nanofiber assembly on the absorber side, the excreted liquid can be diffused at a position farther from the wearer's skin, and this makes it possible to particularly improve the diffusibility and prevent the returning. In addition, such a structure can be easily manufactured only by applying a dispersion liquid of cellulose nanofibers (single-sided application) only to the surface of the nonwoven fabric of the intermediate sheet on the absorber side.

### <Fourth Aspect>

In the absorbent article according to the second or third aspect,
the adhered portion of the cellulose nanofiber assembly has a crosslinking portion in which the cellulose nanofiber assembly crosslinks fibers of the intermediate sheet.

### (Function and Effect)

By having such a crosslinking portion, the diffusibility can be further improved without impairing the permeability.

### <Fifth Aspect>

In the absorbent article according to any one of the first to fourth aspects,
the intermediate sheet is a short fiber nonwoven fabric having a thickness of 0.3 to 1.0 mm, a constituent fiber fineness of 2 to 10 dtex, and a basis weight of 20 to 50 g/m², and
the adhered portion of the cellulose nanofiber assembly contains 2 to 5 g/m² of cellulose nanofiber.

### (Function and Effect)

If the material of the intermediate sheet is such a bulky nonwoven fabric, the top sheet can be separated from the absorber side, and this is preferable because cushioning property and returning prevention can be improved, but the liquid diffusibility in the direction orthogonal to the thickness direction is deteriorated. However, even such a short fiber nonwoven fabric can supplement the liquid diffusibility by having the adhered portion of the cellulose nanofiber assembly as described above.

Incidentally, "thickness" described in this aspect means the thickness when a pressure of 0.196 N/cm² is applied to the nonwoven fabric to be measured by a 2 cm² circular pressure plate in an automated compression tester "KES-G5" made by KATO TECH CO., LTD.

### <Sixth Aspect>

In the absorbent article according to any one of the first to fifth aspects,
the adhered portions of the cellulose nanofiber assembly continuing in an elongated shape are arranged at intervals, over a range of 50% or more of the area of the intermediate sheet, and
the area ratio of the adhered portions of the cellulose nanofiber assembly to the area of the intermediate sheet is 10 to 30%.

### (Function and Effect)

In the adhered portions of the cellulose nanofiber assembly, the diffusibility is improved as the area is increased, but the intermediate sheet is hardened. Therefore, as in the present aspect, when the adhered portions of the cellulose nanofiber assembly are elongated, spaced over a wide range of the intermediate sheet, and arranged at a predetermined area ratio, while improving the diffusibility, the hardening can be suppressed, and the flexible fit and softness of the absorbent article are unlikely to be impaired.

### Advantageous Effects of Invention

According to the present invention, the diffusibility of excreted liquid is improved by a simple method.

### Brief Description of Drawings

Fig. 1 is a plan view illustrating the front surface of a tape-type disposable diaper in a state where a diaper is spread.
Fig. 2 is a plan view illustrating the back surface of a tape-type disposable diaper in a state where a diaper is spread.
Fig. 3 is a cross-sectional view taken along line 6-6 in Fig. 1.
Fig. 4 is a cross-sectional view taken along line 7-7 in Fig. 1.
Fig. 5 is a cross-sectional view taken along line 8-8 in Fig. 1.
Fig. 6 is a cross-sectional view taken along line 9-9 in Fig. 1.
Fig. 7 is a cross-sectional view taken along line 5-5 in Fig. 1.
Fig. 8 is a cross-sectional view illustrating an application form of cellulose nanofibers.
Figs. 9 are cross-sectional views illustrating application forms of cellulose nanofibers.
Fig. 10 is a cross-sectional view illustrating an application form of cellulose nanofibers.
Fig. 11 is a cross-sectional view illustrating an application form of cellulose nanofibers.
Fig. 12 is an explanatory view illustrating the overlapping of sheets.
Figs. 13 are explanatory views illustrating the arrangement of adhered portions of a cellulose nanofiber assembly.
Figs. 14 are explanatory views illustrating the arrangement of adhered portions of a cellulose nanofiber assembly.
Fig. 15 is a cross-sectional photograph of an intermediate sheet provided with an adhered portion of a cellulose nanofiber assembly.
Fig. 16 is a surface photograph of an intermediate sheet provided with an adhered portion of a cellulose nanofiber assembly.

### Description of Embodiments

Figs. 1 to 7 illustrate examples of a tape-type disposable diaper, in which the reference character X indicates the maximum width of the diaper excluding a connecting tape, and the reference character L indicates the maximum length of the diaper. Respective constituent members can be bonded to an adjacent member at an appropriate position. The dotted pattern portion in the cross-sectional view indicates an adhesive as a bonding means. The bonding between members with an adhesive can be performed by, for example, the solid, bead, curtain, summit, or spiral application of a hot melt adhesive, or pattern coating (transfer of the hot melt adhesive in a letterpress method), or the fixing of an elastic member is performed by application on the outer peripheral surface of the elastic member, such as a comb gun or a surewrap application in place of or in addition to the above. Examples of the hot melt adhesive include, but are not limited to, adhesives of an EVA-based, pressure sensitive adhesive rubber-based (elastomer-based), polyolefin-based, and polyester/polyamide-based (however, cellulose nanofibers are not included in the adhesive). As a bonding means for bonding respective constituent members, a means by material welding such as heat sealing or ultrasonic sealing can also be used.

This tape-type disposable diaper includes an absorber 56, a liquid pervious top sheet 30 covering the front surface side of the absorber 56, a liquid impervious resin film 11 covering the back surface side of the absorber 56, and an outer nonwoven fabric 12 that covers the back surface side of the liquid impervious resin film and constitutes the back surface of the product. The reference character F indicates a ventral side portion located on the front side with respect to the center in the front-back direction, and the reference character B indicates a dorsal side portion located on the back side with respect to the center in the front-back direction.

As illustrated in Figs. 1 and 2, the tape-type disposable diaper in this example has a crotch portion A2 and portions A1 and A3 extending on both front and back sides of the crotch portion A2. Here, the crotch portion A2 means a part corresponding to the crotch of the wearer. For example, when the middle width in the front-back direction of the absorber 56 is narrow along the leg circumference, the crotch portion A2 can be the range in the front-back direction of the narrowing part (constricted part). Further, the crotch portion A2 can be, for example, a central part when the absorbent article is divided into three in the front-back direction LD.

The materials and characteristic parts of each portion will be described below in order.

### (Absorber)

The absorber 56 is a part that contains a super absorbent polymer and absorbs and holds body fluids such as excreted liquid and blood. As the absorber 56, a known absorber can be appropriately used, such as an absorber having a structure in which a super absorbent polymer is held by an assembly of fibers, and an absorber having a structure in which a super absorbent polymer is sandwiched between liquid pervious sheets. As the fiber assembly, besides those obtained by stacking short fibers such as fluff pulp and synthetic fibers, a filament assembly obtained by opening tows (fiber bundles) of synthetic fibers such as cellulose acetate as required can also be used. When fluff pulp or short fibers are accumulated, fiber basis weight can be set to, for example, about 100 to 300 g/m², and in the case of a filament assembly, fiber basis weight can be set to about 30 to 120 g/m². In the case of a synthetic fiber, the fineness is, for example, 1 to 16 dtex, preferably 1 to 10 dtex, more preferably 1 to 5 dtex. In the case of filament assembly, the filaments may be non-crimped fibers, but are preferably crimped fibers. The degree of crimp of the crimped fiber can be, for example, about 5 to 75 crimps, preferably about 10 to 50 crimps, and more preferably about 15 to 50 crimps per 2.54 cm. In addition, crimped fibers which are uniformly crimped can be used.

### (Super Absorbent Polymer Particle)

The shape of the super absorbent polymer contained in the absorber 56 is not particularly limited, but is preferably particulate. The super absorbent polymer particle means "powder" in addition to "particle". As the super absorbent polymer particles 54, those used for this type of absorbent articles can be used on an as-is basis. The particle diameter of the super absorbent polymer particles is not particularly limited, but for example, when the particles are sieved (shaking for five minutes) using a standard sieve (JIS Z8801-1: 2006) of 500 µm and the particles subjected to sieving with the 500 µm standard sieve are further sieved (shaking for five minutes) using the standard sieve (JIS Z8801-1: 2006) of 180 µm, desirably the proportion of the particles remaining on the 500 µm standard sieve is 30% by weight or less, and the proportion of the particles remaining on the 180 µm standard sieve is 60% by weight or more. The super absorbent polymer contained in the absorber 56 may be entirely movable, or may be entirely or partially fixed to another material such as the above-described fiber assembly or a package sheet 58 described later.

The material of the super absorbent polymer is not particularly limited, but materials having a water absorption capacity of 30 g/g or more are suitable. Examples of the super absorbent polymer particles include starch-based, cellulose-based, and synthetic polymer-based particles, and starch-acrylic acid (salt) graft copolymers, saponified starch-acrylonitrile copolymers, crosslinked sodium carboxymethylcellulose, and acrylic acid (salt) polymers can be used. As the shape of the super absorbent polymer particles, particulate materials which are usually used are preferable, but other shapes can also be used.

The super absorbent polymer particles having a water absorption speed of 70 seconds or less, particularly 40 seconds or less, are suitably used. If the water absorption speed is too slow, back-flow, in which the liquid fed into the absorber 56 returns to the outside of the absorber 56, is likely to occur.

As the super absorbent polymer particles, those having a gel strength of 1,000 Pa or more are preferably used. Thereby, even when the absorber 56 is bulky, it is possible to effectively suppress stickiness after liquid absorption.

The basis weight of the super absorbent polymer can be appropriately determined according to the absorption amount required for the use of the absorber 56. Therefore, although it cannot be said unconditionally, the basis weight can be 50 to 350 g/m². When the basis weight of the polymer is less than 50 g/m², it is difficult to ensure the absorption amount. If it exceeds 350 g/m², not only the effect is saturated, but also an excessive amount of super absorbent polymer particles causes a gritty and uncomfortable feeling.

In the absorber 56 containing super absorbent polymer particles, when the super absorbent polymer particles absorb excreted liquid and expand, a liquid passage in the absorber shrinks, and due to the shrinkage of this passage, "gel blocking" in which the diffusibility of the liquid in the absorber is lowered is likely to occur. In general, gel blocking is likely to occur in a site where excreted liquid is likely to concentrate, that is, at a crotch portion or a vicinity of the crotch portion. Therefore, it is considered that when gel blocking occurs, the absorption speed decreases, and returning is likely to occur.

### (Package Sheet)

In order to prevent the super absorbent polymer particles from coming off or to improve the shape retention of the absorber 56, the absorber 56 can be incorporated as an absorbent element 50 wrapped with the package sheet 58. As the package sheet 58, a tissue paper, particularly a crepe paper, a nonwoven fabric, a polyethylene laminated nonwoven fabric, a sheet with small holes, and the like can be used. However, it is desirable that the sheet from which the super absorbent polymer particles do not come off is used. When a nonwoven fabric is used in place of a crepe paper, a hydrophilic SMMS (spun bond/melt blown/melt blown/spun bond) nonwoven fabric is particularly suitable, and polypropylene, polyethylene/polypropylene, and the like can be used as the material. The fiber basis weight is desirably 5 to 40 g/m², particularly desirably 10 to 30 g/m².

As illustrated in Fig. 3, the package sheet 58 has a form in which the entire absorber 56 is wrapped with one sheet, or the package sheet 58 in which the entire absorber 56 may be wrapped with a plurality of sheets such as upper and lower two sheets. The package sheet 58 can be omitted.

### (Top Sheet)

The top sheet 30 has a liquid perviousness, and for example, such as a perforated or imperforate nonwoven fabric and a porous plastic sheet can be used. Among them, a raw material fiber of the nonwoven fabric is not particularly limited. Examples of the raw material fiber include synthetic fibers such as polyolefin-based such as polyethylene and polypropylene, polyester-based, and polyamide-based, regenerated fibers such as rayon and cupra, natural fibers such as cotton, and mixed fibers, and composite fibers in which two or more of these are used. Further, the nonwoven fabric may be manufactured by any processing. Examples of the processing method include known methods such as a spun lace method, a spun bond method, a thermal bond method, a meltblown method, a needle punch method, an air through method, and a point bond method. For example, the spun lace method is preferable when flexibility and drapeability are required, and the thermal bond method is preferable when bulkiness and softness are required.

The top sheet 30 extends from the front end to the back end of the product in the front-back direction and extends laterally beyond the absorber 56 in the width direction WD. For example, such as when the starting point of the rising gathers 60, which will be described later, is located closer to the center in the width direction than the side edges of the absorber 56, if necessary, the top sheet 30 can be appropriately deformed such as making the width of the top sheet 30 shorter than the maximum width of the absorber 56.

### (Intermediate Sheet)

In the present tape-type disposable diaper, an intermediate sheet (also referred to as a "second sheet") 40 is provided in order to prevent the above-described "returning" phenomenon.

Examples of the intermediate sheet 40 include the same material as the top sheet 30, a spun lace nonwoven fabric, a spun bond nonwoven fabric, an SMS nonwoven fabric, a pulp nonwoven fabric, a mixed sheet of pulp and rayon, a point bond nonwoven fabric, or a crepe paper. In particular, an air-through nonwoven fabric is preferable because it is bulky. It is preferable to use a composite fiber having a core-sheath structure for the air-through nonwoven fabric. In this case, resin used for the core may be polypropylene (PP), but polyester (PET) having high rigidity is preferable. The basis weight is preferably 17 to 80 g/m², more preferably 25 to 60 g/m². The fineness of the constituent fibers of the nonwoven fabric is preferably 2.0 to 10 dtex, more preferably 1.7 to 5 dtex. Further, long fibers and short fibers can be used as the fibers constituting the nonwoven fabric. In particular, short fibers can be used, and the fiber length is preferably 35 to 60 mm, more preferably 40 to 55 mm. To increase the bulkiness of the nonwoven fabric, it is preferable to use eccentric fibers, hollow fibers, eccentric and hollow fibers, whose core is not in the center, as mixed fibers of all or a part of the raw material fibers. Particularly in the case of the intermediate sheet made of a short fiber nonwoven fabric, the basis weight is preferably 20 to 50 g/m².

As the nonwoven fabric of the intermediate sheet 40, a nonwoven fabric made of raw material fibers having a higher hydrophilicity than the nonwoven fabric of the top sheet 30 may be used, and the same material as the top sheet 30 to which a hydrophilizing agent is added may also be used. The raw material fibers of the nonwoven fabric used for the intermediate sheet 40 are not particularly limited, but synthetic fibers such as polyolefin-based such as polyethylene and polypropylene, polyester-based, polyamide-based and the like can be used. As a method for processing the nonwoven fabric forming the intermediate sheet 40, known methods, for examples, such as a spun lace method, a spun bond method, a thermal bond method, a meltblown method, a needle punch method, an air through method, and a point bond method, can be used, but an air through method bringing about high bulkiness can be preferably used.

The Clark stiffness (JIS P 8143 (2009)) of the nonwoven fabric forming the intermediate sheet 40 is 30 to 80, particularly 30 to 60, and is preferably lower than that of the top sheet 30. When the rigidity is higher than 80, the soft feeling of the intermediate sheet 40 portion of a wearable article is impaired, and when the rigidity is lower than 30, the thickness feeling required for the intermediate sheet 40 is impaired.

The intermediate sheet 40 in the form illustrated in Fig. 12 has the same width as the top sheet 30, but it may be shorter than the width of the absorbent element 50 and arranged only in the center. The length of the intermediate sheet 40 in the front-back direction may be the same as the maximum length of a diaper, may be the same as the length of the absorbent element 50, or may be within a short length range around a region receiving a liquid.

The thickness when a pressure of 0.196 N/cm² is applied to the nonwoven fabric to be measured by a 2 cm² circular pressure plate in an automated compression tester "KES-G5" made by KATO TECH CO., LTD. is preferably in the range of 0.3 to 1 mm, and more preferably in the range of 0.6 to 0.9 mm.

The intermediate sheet 40 is preferably bonded to the top sheet 30. As a bonding method, known methods such as bonding with a hot melt adhesive or the like, spun lace, heat embossing, ultrasonic welding and the like can be used. In particular, heat embossing is preferable because the top sheet 30 becomes uneven and the liquid diffusibility increases. In the uneven top sheet 30, a large number of concave sites are formed on the intermediate sheet 40 side. This makes it difficult for liquid to permeate to the intermediate sheet 40 side only at a specific position on the top sheet 30, and the liquid is diffused into a large number of concaves and is easily permeated.

When heat embossing is used as a bonding means, it is preferable that the material of the intermediate sheet 40 has a melting point similar to that of the top sheet 30. It is preferable that the top sheet 30 and the intermediate sheet 40 are bonded to each other in all the concaves, but they may have unbonded portions.

### (Liquid Impervious Resin Film)

The liquid impervious resin film 11 is not limited as long as it has moisture penetrability, but for example, a microporous sheet can be preferably used which is obtained by kneading an inorganic filler in a polyolefin-based resin such as polyethylene or polypropylene, molding the kneaded resin into a sheet, and stretching the sheet in one or two axial directions. In particular, the liquid impervious resin film 11 may have moisture penetrability in the thickness direction. It is obvious that the liquid impervious resin film 11 does not include one having a nonwoven fabric as a base material and treated to enhance waterproofness.

The liquid impervious resin film 11 desirably extends in the front-back direction LD and the width direction WD in the same range as the absorber 56 or in the wider range than the absorber 56, but if there are other water blocking means, if necessary, it is also possible to adopt a form in which the ends of the absorber 56 are not covered in the front-back direction LD and the width direction WD.

### (Outer Nonwoven Fabric)

The outer nonwoven fabric 12 covers the entire back surface side of the liquid impervious resin film 11 and makes the back surface of the product look like a cloth. The outer nonwoven fabric 12 is not particularly limited. As a raw material fiber, for example, in addition to synthetic fibers such as polyolefin-based such as polyethylene or polypropylene, polyester-based, and polyamide-based, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spun bond nonwoven fabric, an SMS nonwoven fabric, and an SMMS nonwoven fabric are preferable in that both good touch feeling and strength can be compatible. In addition to using a single piece of nonwoven fabric, it is also possible to use multiple nonwoven fabrics in layers. In the latter case, it is preferable that the nonwoven fabrics are adhered to each other with a hot melt adhesive or the like. When a nonwoven fabric is used, the basis weight of the fiber is desirably 10 to 50 g/m², particularly desirably 15 to 30 g/m².

### (Rising Gather)

To prevent excrement that moves laterally on the top sheet 30 and to prevent side leakage, rising gathers 60 that stand up on the wearer's skin side are preferably provided on both sides of the surface in the width direction WD. It is obvious that the rising gather 60 can be omitted.

When the rising gather 60 is adopted, its structure is not particularly limited, and any known structure can be adopted. The rising gather 60 in the illustrated example is composed of a gather sheet 62 that is substantially continuous in the width direction WD, and an elongated gather elastic member 63 that is fixed to the gather sheet 62 in a stretched state along the front-back direction LD. As this gather sheet 62, a water-repellent nonwoven fabric can be used, and rubber thread and the like can be used as the gather elastic member 63. As illustrated in Figs. 1 and 2, one or a plurality of the elastic member can be provided for each.

The front surface of the gather sheet 62 has a bonding start point in the width direction WD on the side portion of the top sheet 30, and the part covering from the bonding start point to the back surface side in the width direction is bonded to the front surface of each side flap portion SF, that is, in the illustrated example, the side portion of the liquid impervious resin film 11 and the side portion of the outer nonwoven fabric 12 located on the back surface side of the liquid impervious resin film 11 in the width direction by a hot melt adhesive or the like.

In the periphery of the legs, the front surface side of the width direction from the bonding start point of the rising gathers 60 is fixed on the top sheet 30 at both ends of the product front-back direction. However, the part therebetween is a non-fixed free portion erected by contraction force of the elastic member 63 to close contact with the body surface.

### (End Flap Portion, Side Flap Portion)

The tape-type disposable diaper illustrated in the example includes a pair of end flap portions EF that do not have the absorber 56 and that extend to the front side and the back side of the absorber 56, respectively, and a pair of side flap portions SF that do not have the absorber 56 and that extend to the sides of both side edges of the absorber 56, respectively.

### (Flat Gather)

A side elastic member 64 made of an elongated elastic member such as a rubber thread is fixed to each side flap portion SF in a state of being extended along the front-back direction LD. As a result, the around-leg portion of each side flap portion SF is configured as a flat gather. The elastic members 64 around the leg portions is provided between the gather sheet 62 and the liquid impervious resin film 11 on the back surface side in the width direction near the bonding start point of the bonded portion of the gather sheet 62 as in the illustrated example, and can also be provided between the liquid impervious resin film 11 and the outer nonwoven fabric 12 in the side flap portion SF. As in the illustrated example, a plurality of the elastic members 64 around the leg portions may be provided on each side, or only one elastic member 64 may be provided on each side.

### (Connecting Tape)

The side flap portion SF of the dorsal part B is provided with a connecting tape 13 that is detachably connected to the back surface of the ventral part F. When a diaper 10 is attached, the connecting tape 13 is turned from both sides of the waist to the back surface of the ventral part F, and a connecting portion 13A of the connecting tape 13 is connected to an appropriate position on the back surface of the ventral part F.

Although the structure of the connecting tape 13 is not particularly limited, in the illustrated example, a sheet base material forming a tape attachment part 13C fixed to the side flap portion SF and a tape main unit section 13B protruding from the tape attachment part 13C, and the connecting portion 13A with respect to the ventral side, which is provided at the intermediate portion in the width direction of the tape main unit section 13B in the sheet base material. A tip end side of the connecting portion 13A is a tab part.

As the connecting portion 13A, a hook material (hook member) of a mechanical fastener (hook and loop fastener) may be provided, or an adhesive layer may be provided. The hook member has a number of engagement projections on its connecting surface, and the engagement projection has (A) a check mark shape, (B) a J shape, (C) a mushroom shape, (D) a T shape, and (E) a double J shape (a shape bonded back to back of a J shape), but may have any shape.

Further, as the sheet base material forming from the tape attachment part 13C to the tape main unit section 13B, nonwoven fabric, plastic film, poly-laminated nonwoven fabric, paper or a composite material of these can be used, but a spun bond nonwoven fabric, an air-through nonwoven fabric, or a spun lace nonwoven fabric, having a fineness of 1.0 to 3.5 dtex and a basis weight of 20 to 100 g/m² is preferable.

### (Target Sheet)

It is preferable to provide a target sheet 20 having a target for facilitating connection at the connecting portion of the connecting tape 13 in the ventral side portion F. In the case where the connecting portion 13A is the hook member, the target sheet 20 can be used in which a large number of loop threads to which an engagement projection of a hook member is tangled are provided on a surface of the sheet base material made of a plastic film or a nonwoven fabric. Further, in the case of an adhesive layer, it is possible to use a sheet base material made of a plastic film having a smooth surface with high adhesiveness and subjected to peel treatment. Further, when the connecting portion of the connecting tape 13 in the ventral part F is made of a nonwoven fabric, for example, when the outer nonwoven fabric 12 is provided as in the illustrated embodiment, the target sheet 20 is omitted and the hook material is entangled and connected as the fiber of the outer nonwoven fabric 12. In this case, the target sheet 20 as a mark may be provided between the outer nonwoven fabric 12 and the liquid impervious resin film 11.

### (Cellulose Nanofiber)

The intermediate sheet 40 has adhered portions 15 of the cellulose nanofiber assembly that continue between at least the crotch portion A2 or a first position in the vicinity of the crotch portion A2 and a second position apart from the first position. Cellulose nanofibers refer to fine cellulose fibers obtained by defibrating pulp fibers and refer to cellulose fibers containing cellulose fine fibers generally having an average fiber width of nanosize (1 nm or more, 1000 nm or less), but the average fiber width (median diameter) is preferably 100 nm or less, and particularly preferably 10 to 60 nm. Further, cellulose fibers are composed of innumerable β-glucose units linked mainly by β-1, 4 glycoside bonds in a chain. β-Glucose has a -H group, a -OH group and the like.

Cellulose nanofibers generally have a fiber width of 4 nm or more and 1000 nm or less, a fiber length of 5 µm or more, a high aspect ratio (5 or more for low and 1250 or more for high), and a large specific surface area. Further, the assembly has high hydrophilicity. Therefore, when the intermediate sheet 40 has the adhered portion 15 of the assembly of cellulose nanofibers, the diffusibility is improved. That is, excreted liquid in contact with the intermediate sheet 40 is promoted so as to diffuse in the direction following the adhered portions 15 of the cellulose nanofiber assembly (that is, from the first position to the second position). Then, the absorption speed is improved due to the improvement of the diffusibility outside the absorber 56, such that the returning phenomenon becomes more difficult to occur. The assembly of cellulose nanofibers may have a porous structure or a three-dimensional network structure containing a large number of pores capable of at least one of air permeability and liquid passage, or may have a dense structure not containing pores, or containing pores although it is not substantially capable of air permeability and liquid passage. It is preferable that the assembly of cellulose nanofibers has a water-absorbing porous structure because the above-described diffusibility is further enhanced.

Actually, as illustrated in Fig. 16, when the adhered portion 15 of the cellulose nanofiber assembly is provided on the intermediate sheet 40, and the edge of the intermediate sheet 40 (the lower portion in Fig. 16) is immersed in water, water begins to permeate the intermediate sheet 40. Although water permeates to the upper portion in the drawing, the present inventors have found that the permeation rate of water to the upper portion of a site where the adhered portion 15 of the cellulose nanofiber assembly is provided is higher than that of a site where the adhered portion 15 of the cellulose nanofiber assembly is not provided.

The arrangement of the adhered portions 15 of the cellulose nanofiber assembly is not particularly limited as long as they continue over the first position and the second position. For example, as long as the first position is at or near the crotch portion, the specific position in the front-back direction and the specific position in the width direction are not limited. Further, the second position may be a position different from the first position at or near the crotch portion, or may be an arbitrary position on at least one side in front of and behind the crotch portion as long as the second position is separated from the first position. The second position may be located at the edge of the intermediate sheet 40 or may be separated from the edge of the intermediate sheet 40.

The distance between the first position and the second position can be appropriately determined in consideration of the improvement of diffusibility, but normally, it is preferably 1/2 or more of the dimension of the intermediate sheet 40 in the width direction or 1/2 or more of the dimension of the crotch portion in the front-back direction. Specifically, the distance between the first position and the second position is preferably 30 mm or more, and particularly preferably 50 mm or more.

Further, the continuation of the adhered portions 15 obviously includes that the adhered portions 15 are continuous without interruption, and also includes the case where the adhered portions continuing (connecting) without interruption of 5 mm or more continue intermittently (in a dotted line) at intervals of 5 mm or less. If the interval is narrow as described above, even if the adhered portions 15 are intermittent, liquid is transferred from the adhered portion to the adhered portion, and the diffusibility is improved as compared with the case where there is no adhered portion.

Obviously, the top sheet 30, the intermediate sheet 40, and the absorber 56 are provided at least in a region from the first position to the second position. However, as described above, the top sheet 30, the intermediate sheet 40, and the absorber 56 may have different dimensions or the same dimensions as long as they each extend over the first position and the second position.

For example, as illustrated in Fig. 13(a), it is preferable that the adhered portions 15 be formed by arranging the adhered portions of a linear cellulose nanofiber assembly in an oblique lattice pattern. It is also preferable to modify this form to form an intermittent form in which the adhered portions of the cellulose nanofiber assembly are not provided at the intersections of the lattices as illustrated in Fig. 13(b) (in this case, the adhered portions are arranged intermittently at intervals of 5 mm or less as described above). In the case of the intermittent form, the amount of the adhered portions 15 formed in the cellulose nanofiber assembly can be reduced, the intermediate sheet 40 can be prevented from being excessively hardened, and the flexibility can be maintained. When the adhered portions 15 of the cellulose nanofiber assembly have a lattice pattern, the line width of the adhered portion 15 of the cellulose nanofiber assembly is preferably 1.0 to 4.0 mm, and particularly preferably 2.0 to 3.0 mm. Further, the interval (in the case of the lattice pattern, intervals between the parallel adhered portions 15 of the cellulose nanofiber assembly) 15d between the adhered portions 15 of the cellulose nanofiber assembly is preferably 5 to 30 mm, and particularly preferably 10 to 20 mm.

Note that when a plurality of the adhered portions 15 is provided, other adhered portions 15 may not satisfy this condition as long as at least one adhered portion 15 continues over the first position and the second position. For example, as for the other adhered portions 15, as illustrated in Figs. 13(c) and 13(d), the adhered portions 15 of a dot-shaped cellulose nanofiber assembly such as circles and short lines may be provided in a staggered pattern such as a zig zag arrangement (illustration example), a matrix arrangement (not illustrated), or the like. When the adhered portions 15 of the cellulose nanofiber assembly has a dotted pattern, the diameter (length of the longest portion) of the adhered portion 15 of the cellulose nanofiber assembly is preferably 1.0 to 4.0 mm, and particularly preferably 2.0 to 3.0 mm. Further, the interval (in the case of the dotted pattern, the interval in the width direction WD and the front-back direction LD) 15d between the adhered portions 15 of the cellulose nanofiber assembly is preferably 5 to 30 mm, and particularly preferably 10 to 20 mm.

The adhered portions 15 of the cellulose nanofiber assembly may be, as illustrated in Fig. 14(c), arranged in vertical stripes in which a plurality of the linear adhered portions 15 along the front-back direction LD are arranged at intervals in the width direction, or may be arranged in horizontal stripes in which a plurality of the linear adhered portions 15 along the width direction WD are arranged at intervals in the front-back direction. Further, as illustrated in Fig. 14(d), a lattice-like arrangement formed of the linear adhered portions 15 of the cellulose nanofiber assembly along the front-back direction LD and the linear adhered portion 15 of the cellulose nanofiber assembly along the width direction WD can be adopted.

The line width of the adhered portion 15 of the cellulose nanofiber assembly is preferably 1.0 to 4.0 mm, and particularly preferably 2.0 to 3.0 mm. The interval between the adhered portions 15 of the cellulose nanofiber assembly is preferably 5 to 30 mm, and particularly preferably 10 to 20 mm.

Further, it is also preferable to arrange the adhered portions 15 of the cellulose nanofiber assembly in a wavy line as illustrated in Fig. 14(b). The mesh shape illustrated in Fig. 14(a) in which the wavy lines are densely arranged is also preferable.

Further, it can be seen from Fig. 16 that, in the adhered portion 15 of the cellulose nanofiber assembly provided on the surface of the intermediate sheet 40, the fibers of the intermediate sheet 40 are crosslinked by the adhered portion 15 of the cellulose nanofiber assembly. When water molecules are adsorbed to the edge of the adhered portion 15 of the cellulose nanofiber assembly, they penetrate in the upward direction in the drawing through the crosslinking adhered portions 15 of the cellulose nanofiber assembly. Note that the cross-linking by the adhered portions 15 of the cellulose nanofiber assembly is not limited to the surface of the intermediate sheet 40, but occurs inside the fibers forming the intermediate sheet 40.

The adhered portion 15 of the cellulose nanofibers can be produced by a known method in which the cellulose nanofiber dispersion liquid dispersed in water or the like is applied to the target sheet, that is, the intermediate sheet 40 in this case, and then dried. Note that, when the cellulose nanofiber dispersion liquid is applied to a fiber sheet such as paper or nonwoven fabric in the production method in which the cellulose nanofiber dispersion liquid is applied as described above, most of the cellulose nanofiber dispersion liquid is deposited on the surface of the sheet, and the rest penetrates the fibers inside the sheet.

The cellulose nanofiber dispersion liquid is, for example, formed by dispersing cellulose nanofibers in a liquid such as water. The concentration (mass/volume) of the cellulose nanofiber dispersion is preferably 0.1 to 10%, more preferably 1.0 to 5.0%, and particularly preferably 1.5 to 3.0%.

The B-type viscosity (60 rpm, 20°C) of the cellulose nanofiber dispersion liquid is, for example, 700 cps or less, preferably 200 cps or less, more preferably 50 cps or less. By thus suppressing the B-type viscosity of the cellulose nanofiber dispersion liquid to a low level, the cellulose nanofibers are uniformly applied to the sheet surface, and the surface property of the sheet is improved uniformly.

To apply the cellulose nanofiber dispersion liquid, a transfer method such as a letterpress method can be used in addition to spraying on the target surface.

Here, a method for measuring the average fiber width of cellulose nanofibers will be described.

First, 100 ml of a cellulose nanofiber aqueous dispersion liquid having a solid content concentration of 0.01 to 0.1% by mass is filtered through a Teflon (registered trademark) membrane filter, and the solvent substitution are performed once with 100 ml of ethanol and three times with 20 ml of t-butanol.

Next, it is freeze-dried and coated with osmium to obtain a sample. This sample is observed with an electron microscope SEM image at a magnification of 5000 times, 10000 times or 30000 times (in this example, a magnification of 30000 times) depending on the width of the constituent fibers. Specifically, two diagonal lines are drawn on the observed image, and three straight lines passing through the intersections of the diagonal lines are drawn arbitrarily. Further, a total of 100 fiber rods intersecting with these three straight lines are visually measured. The median diameter of the measured values is taken as the average fiber width.

Examples of pulp fibers that can be used to produce cellulose nanofibers include chemical pulp such as hardwood pulp (LBKP) and softwood pulp (NBKP), mechanical pulp such as bleached thermomechanical pulp (BTMP), stone ground pulp (SGP), pressure stone ground pulp (PGW), refiner ground pulp (RGP), chemi ground pulp (CGP), thermo ground pulp (TGP), ground pulp (GP), thermo mechanical pulp (TMP), chemi-thermo mechanical pulp (CTMP), and refiner mechanical pulp (RMP), waste pulp produced from such as waste tea paper, craft envelope blind paper, magazine waste paper, newspaper waste paper, flyer waste paper, office waste paper, corrugated waste paper, Kamishiro waste paper, Kent waste paper, imitation waste paper, land voucher waste paper, and woody waste paper, and deinked pulp (DIP) obtained by deinking waste paper pulp. These may be used alone or in combination of two or more kinds as long as the effects of the present invention are not impaired. Furthermore, the pulp fibers that have been chemically treated such as carboxymethylation may be used.

Examples of the method for producing cellulose nanofibers include mechanical methods such as a highpressure homogenizer method, a microfluidizer method, a grinder grinding method, a bead mill freeze-grinding method, and an ultrasonic defibration method, but are not limited to these methods. Further, the nanofiber formation is promoted by the combined use of TEMPO oxidation treatment, phosphoric acid esterification treatment, acid treatment and the like.

As long as the adhered portion 15 of the cellulose nanofiber assembly is adhered to at least a part of the thickness direction of the intermediate sheet 40, it may be adhered only to a part of the thickness direction, and may be adhered to the entire thickness direction. When the adhered portion 15 of the cellulose nanofiber assembly is exposed on at least one of the front and back surfaces of the intermediate sheet 40, it is preferable that the liquid diffusibility on the top sheet 30 side or the absorber 56 side be improved. In this case, the adhered portion 15 may hardly exist inside the intermediate sheet 40, or may fully exist up to the middle in the thickness direction.

The structure in which the adhered portion 15 of the cellulose nanofiber assembly is exposed on at least one of the front and back surfaces of the intermediate sheet 40 can be formed by applying the cellulose nanofiber dispersion liquid to at least one of the front and back surfaces of the intermediate sheet 40 and then drying, as described above. For example, when the cellulose nanofiber dispersion is applied to the surface 40e of the intermediate sheet 40, usually, not only the coated surface, but also the fibers inside the intermediate sheet 40 are infiltrated (permeated) with the same dispersion liquid, and the adhered portion 15 of the cellulose nanofiber assembly is formed. The distribution of the cellulose nanofiber assembly in the thickness direction of the adhered portion 15 can be adjusted by the depth of penetration into the fiber. For example, as illustrated in Fig. 8, the adhered portion 15 of the cellulose nanofiber assembly may be formed while maintaining a predetermined width from the front surface 40e of the intermediate sheet to the back surface 40f. Further, as illustrated in Fig. 9(a), the content of the adhered portion 15 of the cellulose nanofiber assembly may change from the front surface 40e of the intermediate sheet toward the back surface 40f. Further, as illustrated in Fig. 10, the adhered portion 15 of the cellulose nanofiber assembly may be provided only on the surface 40e side in the thickness direction of the intermediate sheet 40 (not exposed on the back surface 40f). As illustrated in Fig. 11, the adhered portion 15 of the cellulose nanofiber assembly may be provided only near the front surface 40e of the intermediate sheet 40. Note that, in Figs. 8 to 11, the adhered portions 15 of the cellulose nanofiber assembly are represented by a dot pattern.

In particular, it is preferable that, on the back surface and inside of the intermediate sheet 40, the cellulose nanofiber assembly be adhered, and the content of the cellulose nanofiber assembly in the intermediate sheet 40 decrease from the absorber 56 side in the intermediate sheet 40 toward the top sheet 30 side. As a result, it is possible to suppress the hardening of the wearer's touch feeling due to the cellulose nanofiber assembly. Further, by relatively increasing the content of the cellulose nanofiber assembly on the absorber side, the excreted liquid can be diffused at a position farther from the wearer's skin, and this makes it possible to particularly improve the diffusibility and prevent the returning. In addition, such a structure can be easily produced by applying the cellulose nanofiber dispersion liquid (single-sided application) only to the back surface of the nonwoven fabric of the intermediate sheet. In particular, it is preferable that the adhered portion 15 of the cellulose nanofiber assembly be provided only on the back surface 40f side of the intermediate sheet 40 in the thickness direction (not exposed on the front surface 40e).

In order to improve the liquid perviousness and liquid diffusibility of the intermediate sheet 40, a large amount of cellulose nanofibers may be better than less amount, but if the amount is excessively large, the product becomes unnecessarily hard. Therefore, the content of cellulose nanofibers is preferably about 2 to 7 g/m². In particular, the content is more preferably about 2 to 5 g/m². Within this range, sufficient liquid perviousness and liquid diffusibility can be secured as a product.

When the adhered portions of the cellulose nanofiber assembly that continue in an elongated shape are arranged on the intermediate sheet, it is preferable to arrange them at intervals over 50% or more of the area of the intermediate sheet. Further, the area ratio defined by the area of the adhered portions of the cellulose nanofiber assembly occupying the area of the intermediate sheet is preferably 10 to 30%. In this manner, it is possible to suppress the hardening while improving the diffusibility, and the flexible fit and softness of the absorbent article are unlikely to be impaired.

The nonwoven fabrics are suitable as the intermediate sheet 40, and in this case, the adhered portion of the cellulose nanofiber assembly has a coated fiber in which the fiber surface of the nonwoven fabric is coated with the cellulose nanofiber assembly, and a gap between the coated fibers, such that, without impairing the liquid perviousness, the diffusibility can be improved microscopically along individual fibers and macroscopically in the direction in which the adhered portions continues. In particular, the high density and high hydrophilicity of the surface of each coated fiber improve the diffusibility of excreted liquid along the fiber.

The intermediate sheet 40 can be used in any material such as a hydrophilic material, a hydrophobic material, and a mixed material of hydrophilicity and hydrophobicity. In particular, it is preferable to provide the adhered portion 15 of the cellulose nanofiber assembly on the intermediate sheet 40 using a hydrophilic material because the fiber is firmly coated with the adhered portion 15.

Fig. 15 is a cross-sectional photograph of the intermediate sheet 40 provided with the adhered portion 15 of a cellulose nanofiber assembly. This intermediate sheet 40 is obtained by applying the cellulose nanofiber dispersion liquid on the surface 40e side, drying it, and then cutting it in the thickness direction at a position passing through the adhered portion 15 of the formed cellulose nanofiber assembly. The cellulose nanofiber assembly reaches the inside of the intermediate sheet 40 and coats the internal fibers of the intermediate sheet 40. In Fig. 15, the coated portions by the cellulose nanofiber assembly is within a range 15G surrounded by a broken line and is portions where the fibers are black. Fig. 16 is a photograph of a sample in which the adhered portion 15 of the cellulose nanofiber assembly is provided on the surface of the intermediate sheet 40 made of a nonwoven fabric.
The adhered portions 15 of the cellulose nanofiber assembly are arranged in the upper and lower portions in the drawing to crosslink the fibers on the front surface of the intermediate sheet 40. By having such a crosslinking portion, the diffusibility can be further improved without impairing the permeability.

### <Others>

The adhered portion 15 of the cellulose nanofiber assembly can be provided not only in the intermediate sheet 40 but also between the liquid impervious resin film 11 and the outer nonwoven fabric 12, over the entire surface or at several positions at intervals. The liquid impervious resin film 11 and the outer nonwoven fabric 12 are paths through which the moisture of the excreted liquid absorbed by the absorber 56 passes toward the outside. Since the adhered portion 15 of the cellulose nanofiber assembly has moisture penetrability, by interposing the adhered portion 15 of the cellulose nanofiber assembly between the liquid impervious resin film 11 and the outer nonwoven fabric 12, the product has excellent moisture penetrability, which is preferable. Further, cellulose nanofibers have a large number of -OH groups and have high hygroscopicity. Due to the high hygroscopicity of cellulose nanofibers, moisture is retained in the adhered portion of the cellulose nanofiber assembly rather than an outer nonwoven fabric that forms a product back surface and an underwear, and the product back surface or underwear are unlikely to have a damp feel.

On the other hand, from the viewpoint of the amount of moisture absorption, it is preferable that the adhered portion 15 of the cellulose nanofiber assembly be wide and be continuously provided.

In this case, it is preferable that the outer nonwoven fabric 12 be a water-repellent nonwoven fabric, because the hygroscopicity of the adhered portion 15 of the cellulose nanofiber assembly increase. The water-repellent nonwoven fabric can be manufactured by adding a water repellent agent to the nonwoven fabric (either internal addition or external addition may be used) by a known method. As the water repellent agent, such as silicone based, paraffin based, and alkyl chromic chloride based water repellent agent can be used.

The adhered portion 15 of the cellulose nanofiber assembly can be formed on the front surface of the outer nonwoven fabric 12, or also a sheet of nonwoven fabric or paper on which the adhered portion 15 of the cellulose nanofiber assembly can be arranged between the liquid impervious resin film 11 and the outer nonwoven fabric 12, but it is preferable to be formed on the back surface of the liquid impervious resin film 11. In particular, since the liquid impervious resin film 11 does not have hygroscopicity, the outer nonwoven fabric 12 tends to have a damp feel, but when the adhered portion 15 of the cellulose nanofiber assembly is provided on the back surface of the liquid impervious resin film 11, it is difficult for the outer nonwoven fabric 12 to have a damp feel. Further, when the adhered portion 15 of the cellulose nanofiber assembly is formed on the liquid impervious resin film 11 made of a moisture penetrable resin film, since the adhered portion 15 of the cellulose nanofiber assembly has a film shape, the liquid impervious resin film 11 also has the advantages that the water impermeability and strength are improved, and the elongation is reduced. Particularly, the liquid impervious resin film 11 made of the moisture penetrable resin film may be subjected to intermittent decorative printing of product logos, pictures of characters, photos or the like that is placed on either or both of the front and back of the product, and in addition to continuous decorative printing of many constituent units such as characters that repeat regularly in the front-back direction LD and width direction WD (size, brand name, maker name, design name, etc.), picture or the like. However, when such decorative printing is performed, it is desirable that the liquid impervious resin film 11 have a small elongation.

### <Effect Confirmation Test>

Tape-type disposable diapers having the structures illustrated in Figs. 1 to 7 were manufactured with various specifications in Table 1, and the following absorption speed test, returning amount test, and thickness measurement were performed. Specifications not described in the table and below are common to all cases.

The adhered portions of the cellulose nanofiber assembly were provided on the front surface of the intermediate sheet in the coating patterns illustrated in Figs. 14(c) and 13(a). The application amount of the cellulose nanofiber dispersion on the surface area of the intermediate sheet was 3.57 g/m². Note that the concentration of the cellulose nanofiber dispersion liquid used for application was 2% (mass/volume) with water as a solvent.

### (Absorption Rate Test)

An absorption speed test was performed as follows.
(1) A sample was spread on a horizontally arranged flat plate and fixed by sticking it such that the top sheet faced up.
(2) An injection cylinder carrying a weight of 2 kg was placed at the center of the sample in the width direction and at the center in the front-back direction. The absorption time (second) until 50 g of artificial urine was completely absorbed from the injection cylinder was determined as the absorptionspeed. This was taken as the first absorptionspeed. After being left for 30 minutes, 50 g of artificial urine was injected again, and the absorption speed until complete absorption was determined. This was taken as the second absorptionspeed. After being left for 30 minutes, 50 g of artificial urine was injected once again, and the absorption speed until complete absorption was determined. This was taken as the third absorptionspeed. Note that whether or not the artificial urine was completely absorbed was determined by visual observation by an operator.

### (Returning Amount Test)

(1) (1) and (2) were performed in the absorption speed test in order, and after 20 minutes had elapsed from the time when the third artificial urine was completely absorbed, a weight of 5 kg was placed at the place where the artificial urine was injected and left for 10 minutes.
(2) After (1) above, twenty pieces of filter paper were placed on the portion where the artificial urine was injected, and a weight of 5 kg (area of the filter paper contact surface 100 cm²) was placed on it and left for 20 seconds.
(3) The weight of the filter paper before and after the test operation of the above (2) was measured, and the weight obtained by subtracting the filter paper weight before the test from the filter paper weight after the test was taken as the returning amount.

### (Thickness Measurement)

(1) The intermediate sheet to be measured is set at the measurement position of an automated compression tester "KES-G5" made by KATO TECH CO., LTD. Then, the thickness (mm) was measured when a pressure of 0.196 N/cm² was applied to the intermediate sheet with a circular pressure plate of 2 cm².

**[Table 1]**

| SAMPLE NUMBER | | | 1 | 2 | | | 3 | 4 | | 5 | | | 6 | | 7 | | 8 | |
|---|---|---|---|---|---|---|---|---|---|---|---|---|---|---|---|---|---|---|
| TOP SHEET PROCESSING/BASIS WEIGHT | | | SPUN BOND 18g/m² | SPUN BOND 18g/m² | | AIR THROUGH 20g/m² | | AIR THROUGH 20g/m² | | AIR THROUGH 20g/m² | | | AIR THROUGH 20g/m² | | AIR THROUGH 20g/m² | | AIR THROUGH 20g/m² | |
| INTERMEDIATE SHEET PROCESSING/BASIS WEIGHT | | | POINT BOND 17 g/m² | CHEMICAL BOND 45g/m² | | CHEMICAL BOND 45g/m² | | AIR THROUGH 40g/m² | | AIR THROUGH 40 g/m² | | | AIR THROUGH 30g/m² | | AIR THROUGH 30g/m² | | AIR THROUGH 35g/m² | |
| | THICKNESS | mm | 0.15 | | | 0.63 | | | 0.06 | | 0.72 | | 0. | 29 | | 0.77 | | 0.84 |
| ADHERED PORTION OF CELLULOSE NANOFIBER ASSEMBLY | | | NOT PROVIDED | NOT PROVIDED | PROVIDED | NOT PROVIDED | PROVIDED | NOT PROVIDED | PROVIDED | NOT PROVIDED | PROVIDED | PROVIDED | NOT PROVIDED | PROVIDED | NOT PROVIDED | PROVIDED | NOT PROVIDED | PROVIDED |
| | PATTERN | | - | - | STRIPE | - | STRIPE | - | STRIPE | - | STRIPE | LATTICE | - | STRIPE | - | STRIPE | - | STRIPE |
| | AREA RATIO | % | - | - | 10 | - | 10 | - | 10 | - | 10 | 30 | - | 10 | - | 10 | - | 10 |
| | | UNIT | - | - | - | - | - | - | - | - | - | - | - | - | - | - | - | - |
| ABSORPTION SPEED | FIRST TIME | SECONDS | 58 | 34 | 32 | 32 | 23 | 33 | 37 | 25 | 23 | 25 | 65 | 72 | 34 | 32 | 33 | 31 |
| | SECOND TIME | SECONDS | 81 | 39 | 34 | 36 | 29 | 38 | 51 | 31 | 26 | 26 | 85 | 104 | 44 | 35 | 53 | 41 |
| | THIRD TIME | SECONDS | 90 | 40 | 35 | 39 | 29 | 43 | 57 | 37 | 30 | 27 | 98 | 141 | 45 | 40 | 60 | 45 |
| RETURNING AMOUNT | | g | 0.48 | 0.14 | 0.60 | 0.36 | 0.37 | 1.10 | 1.27 | 0.51 | 0.64 | 0.62 | 1.43 | 1.67 | 1.17 | 1.41 | 0.57 | 0.65 |

The absorption speed test and returning rate test are indicated in Table 1. Further, the thickness of the intermediate sheet is indicated in the table. For sample numbers 2, 3, 5, 7, and 8, the test result was obtained that the absorption speed of the cellulose nanofiber assembly provided with the adhered portion was higher than that of the cellulose nanofiber assembly not provided with the adhered portion.

For sample numbers 4 and 6, the test result was not obtained that the absorption speed of the cellulose nanofiber assembly provided with the adhered portion was higher than that of the cellulose nanofiber assembly not provided with the adhered portion. The intermediate sheets of sample numbers 4 and 6 were thinner than 0.30 mm.

### <Explanation of Terms Used Herein>

In the case where the following terms are used in the specification, those have the following meanings unless otherwise specified in the specification.

"Front-back (longitudinal) direction" means a direction connecting the ventral side (front side) and the dorsal side (back side). "Width direction" means a direction orthogonal to the front-back direction (right-left direction).

"Front surface side" means the side closer to the wearer's skin, and "back surface side" means the side far from the wearer's skin. The "front surface" means the surface of the member that is closer to the wearer's skin, and the "back surface" means the surface of the member that is farther from the wearer's skin.

"LD direction" and "WD direction" mean the flow direction (LD direction) in a manufacturing equipment and the lateral direction (WD direction) orthogonal to the flow direction, and either one is the front-back direction of a product, and the other is the width direction of the product. The LD direction of a nonwoven fabric is the direction of fiber orientation of the nonwoven fabric. "Fiber orientation" is a direction along which a fiber of a nonwoven fabric runs and determined by, for example, a measurement method in accordance with the fiber orientation test method based on the zero distance tensile strength of TAPPI standard method T481 and a simple measurement method for determining the fiber orientation direction from the tensile strength ratio in the front-back direction and the width direction.

"Unfolded state" means a flatly spread state without shrinkage or slackness.

"Stretch rate" means the value when the natural length is taken as 100%.

"Gel strength" is measured as follows: A super absorbent polymer 1.0 g is added to artificial urine (urea: 2wt%, sodium chloride: 0.8wt%, calcium chloride dihydrate: 0.03wt%, magnesium sulfate heptahydrate: 0.08wt%, and ion exchanged water: 97.09wt%) 49.0 g, and stirred with a stirrer. After leaving generated gel for three hours in a thermo-hygrostat bath at 40°C x 60%RH, and returning to room temperature, the gel strength is measured with a curdmeter (Curdmeter-MAX ME-500, made by I. techno Engineering).

"Basis weight" is measured as follows. After preliminary drying a sample or a test piece, the sample or the test piece is left in a test chamber or equipment in the standard state (the test location is at a temperature of 23 ± 1°C and with a relative humidity of 50 ± 2%) to be constant weight. The preliminary drying refers to making a sample or a test piece a constant weight in an environment at a temperature of 100°C. For fibers with an official moisture regain of 0.0%, preliminary drying may not be performed. A sample of dimensions of 100 mm × 100 mm is cut using a template for sampling (100 mm × 100 mm) from a test piece in a constant weight. The basis weight is set by weighing the sample, multiplying by 100, and calculating the weight per one square meter.

"Water absorption capacity" is measured according to JIS K7223-1996 "Testing method for water absorption capacity of super absorbent polymers".

"Thickness" is automatically measured under the conditions of a load of 0.196 N/cm² and a pressing area of 2 cm² using an automatic thickness measuring device (KES-G5 handy compression testing machine).

When environmental conditions in tests and measurements are not described, the tests and measurements shall be carried out in a test room or apparatus in a standard state (a temperature of 23 ± 1°C and a relative humidity of 50 ± 2% at the test location).

The dimension of each portion means the dimension in the unfolded state, not the natural length state, unless otherwise stated.

The invention made by the inventors has been described above as an embodiment. However, the present invention should not be limited by the description and drawings describing the present embodiment. Other examples and the like performed by those skilled in the art based on the present embodiment are included in the present invention.

### Industrial Applicability

The present invention can be applied to disposable diapers in general, such as tape-type disposable diapers as in the above example, underpants-type disposable diapers and pad-type disposable diapers, and it is obvious that the present invention can also be applied to other absorbent articles such as sanitary napkins.

### Reference Signs List

- 11: liquid impervious resin film
- 12: outer nonwoven fabric
- 13: connecting tape
- 13A: connecting portion
- 13B: tape main unit section
- 13C: tape attachment part
- 15: adhered portion of cellulose nanofiber assembly
- 20: target sheet
- 21: edge portion
- 30: top sheet
- 40: intermediate sheet
- 50: absorbent element
- 56: absorber
- 56W: absorber width
- 58: package sheet
- 60: rising gather
- 62: gather sheet
- B: dorsal part
- F: ventral part
- WD: width direction
- LD: front-back direction

## Claims

1. An absorbent article, comprising:
a liquid pervious top sheet including a portion located on a front surface;
an absorber containing a super absorbent polymer provided on a back surface side of the top sheet; and
a liquid pervious intermediate sheet provided between the top sheet and the absorber,
wherein which the top sheet, the intermediate sheet, and the absorber are provided at least in a region from a first position in a crotch portion or in a vicinity of the crotch portion to a second position apart from the first position,
the intermediate sheet has an adhered portion of a cellulose nanofiber assembly continuing at least from the first position to the second position.

2. The absorbent article according to claim 1,
wherein the intermediate sheet is a nonwoven fabric, and the adhered portion of the cellulose nanofiber assembly includes coated fibers, in which a fiber surface of a nonwoven fabric is coated with the cellulose nanofiber assembly, and a gap between the coated fibers.

3. The absorbent article according to claim 2,
wherein the intermediate sheet contains the cellulose nanofiber assembly on the surface of the absorber side and inside the absorber, and
the content of the cellulose nanofiber assembly in the intermediate sheet is decreasing from the absorber side in the intermediate sheet toward the top sheet side.

4. The absorbent article according to claim 2 or 3,
wherein the adhered portion of the cellulose nanofiber assembly has a crosslinking portion in which the cellulose nanofiber assembly crosslinks fibers of the intermediate sheet.

5. The absorbent article according to any one of claims 1 to 4,
wherein the intermediate sheet is a short fiber nonwoven fabric having a thickness of 0.3 to 1.0 mm, a constituent fiber fineness of 2 to 10 dtex, and a basis weight of 20 to 50 g/m², and
the adhered portion of the cellulose nanofiber assembly contains 2 to 5 g/m² of cellulose nanofiber.

6. The absorbent article according to any one of claims 1 to 5,
wherein the adhered portions of the cellulose nanofiber assembly continuing in an elongated shape are arranged at intervals, over a range of 50% or more of the area of the intermediate sheet, and
the area ratio of the adhered portion of the cellulose nanofiber assembly to the area of the intermediate sheet is 10 to 30%.
